(19) **Europäisches Patentamt / European Patent Office / Office européen des brevets**

(11) **EP 2 246 700 A1**

(12) **EUROPEAN PATENT APPLICATION**

(43) Date of publication:
**03.11.2010 Bulletin 2010/44**

(51) Int Cl.:
***G01N 33/50*** (2006.01)

(21) Application number: **10008288.2**

(22) Date of filing: **04.01.2007**

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.01.2006 US 756301 P**

(62) Document number(s) of the earlier application(s) in accordance with Art. 76 EPC:
**07717805.1 / 1 977 241**

(71) Applicant: **Novartis AG**
**4056 Basel (CH)**

(72) Inventors:
• **Kunich, John**
**Emeryville, California 94608 (US)**

• **Liu, Cheng**
**Richmond, California 94803 (US)**

(74) Representative: **Marshall, Cameron John**
**Carpmaels & Ransford**
**One Southampton Row**
**London**
**WC1B 5HA (GB)**

Remarks:
This application was filed on 09-08-2010 as a divisional application to the application mentioned under INID code 62.

(54) **Antibody-dependent cellular cytotoxicity assay**

(57) Methods for detecting antibody dependent cellular cytotoxicity (ADCC) are described herein. The methods are label-free, and can be performed in real time on adherent cells. The methods can include, for example, (a) monitoring the impedance between electrodes on a non-conducting substrate that supports the growth of target cells in an assay medium; and (b) adding effector cells and an antibody that binds to the target cells to the assay medium; wherein any decrease in the impedance between the electrodes on the substrate following addition of the effector cells and the antibody is indicative of ADCC function having been effected in the assay medium.

**Figure 5**

EP 2 246 700 A1

**Description**

**CROSS-REFERENCE TO RELATED APPLICATIONS**

This application claims the benefit of priority from U.S. Provisional Application Serial No. 60/756,301, filed January 4, 2006.

**TECHNICAL FIELD**

The present application relates to methods for assaying antibody-dependent cellular cytotoxicity (ADCC) and, in some embodiments, relates to methods that allows for ADCC to be assayed in real-time without the need for labeled cells.

**BACKGROUND**

ADCC is a component of the immune response in which IgG antibodies bind to antigens on the surface of pathogenic or tumorigenic target cells, which identifies them for destruction by NK effector cells. Effector cells bearing the Fc gamma receptor (FcγR) recognize and bind the Fc region of the antibodies bound to the target cell. The antibodies thus confer specificity to the target cell killing, which is mediated by effector cells forming a bridge between the two cell types and subsequently releasing lytic granules.

Some therapeutic antibodies achieve their biological activities by promoting ADCC. For example, Herceptin®, a humanized antibody used for treatment of breast cancer, promotes ADCC by binding the HER-2 antigen on the surface of cancer cells. Likewise, Rituxan®, a chimeric antibody used for treatment of non-Hodgkin's lymphoma, promotes ADCC by binding CD20 antigens on the surface of lymphoma cells.

Technologies for determining whether an antibody induces ADCC typically involve labeling target cells with a radioactive material, such as $Cr^{51}$, or a fluorescent dye, such as Calcein AM. The labeled cells are incubated with the antibody and effector cells such as NK cells or PBMCs, and killing of the target cells by ADCC is detected by the release of radioactivity or fluorescence. The labeling of target cells in such assays requires detachment of adherent cells for labeling. After labeling, the assay is carried out with the target cells in suspension, which is not a normal state for adherent cells. Furthermore, the extent of cell killing mediated by ADCC generally is determined at only one time point several hours after the mixing of the target cells with effector cells and the antibody. A label-free assay has been developed, in which cell killing is determined by measuring release of lactate dehydrogenase (LDH) from the cytoplasm of lysed cells into the cell-free supernatant. However, the LDH method is not a real-time assay, and it does not distinguish between death of target cells and death of effector cells since both types of cells will release LDH on lysis.

In view of the number of therapeutic antibody products that are now on the market or in development, there is a need for *in vitro* assays to allow the ADCC activity of antibodies to be determined. In particular, there is a need for a high-throughput, label-free ADCC assay that allows ADCC to be monitored in real-time and that does not require detachment of adherent cells.

**SUMMARY**

The present application provides methods of assaying ADCC *in vitro.* methods are label-free or require reduced levels of label, and thus are easier and safer to perform than assays requiring labeled cells. In some embodiments the methods also are performed on adherent cells, rather than on cells in suspension. In addition, the methods can be performed in real-time, allowing the kinetics of ADCC reactions to be followed so that the ADCC kinetics of different antibodies can be compared. The methods provided herein also are more sensitive than standard ADCC assays, allowing minor differences in the ADCC kinetics of different antibodies to be detected. Further, in some embodiments the simplicity of the methods provided herein means that they are faster than standard ADCC assays, and also have the potential to be optimized for high-throughput screening of ADCC activity.

The methods provided herein comprise (a) monitoring the impedance between electrodes on a non-conducting substrate which supports the growth of target cells in an assay medium; and (b) adding to the assay medium effector cells and an antibody that binds to the target cells. A decrease in the impedance between the electrodes on the substrate following addition of the effector cells and the antibody is indicative of ADCC having been effected in the assay medium. An increase or no change in the impedance between the electrodes on the substrate following addition of the effector cells and the antibody can be indicative of ADCC function not having been effected in the assay medium.

In one aspect, the present application features a method of assaying antibody-dependent cellular cytotoxicity (ADCC), comprising: (a) monitoring the impedance between electrodes on a non-conducting substrate that supports the growth of target cells in an assay medium; and (b) adding to the assay medium effector cells and an antibody that binds to the target cells; wherein a decrease in the impedance between the electrodes on the substrate following addition of the effector cells and the antibody is indicative of ADCC function having been effected in the assay medium, and wherein an increase or no change in the impedance between the electrodes on the substrate following addition of the effector cells and the antibody is indicative of ADCC function not having been effected in the assay medium. The method can include measuring impedance at regular intervals. The can include deriving a Cell Index (CI) from each impedance measurement and determining whether a change in Cell Index occurs, wherein the Cell Index is derived from each impedance measurement using the formula

$$CI = \max_{i=1,..,N}\left( \frac{R_{cell}(f_i)}{R_b(f_i)} - 1 \right)$$

wherein $R_b(f)$ and $R_{cell}(f)$ are the frequency-dependent electrode resistances without cells or with cells present, respectively, and N is the number of the frequency points at which the impedance is measured. The method can be conducted using the RT-CES® system. The impedance measurements can be taken every 15 minutes.

The method can further plating the target cells. The target cells can be plated 18 to 24 hours prior to addition of the antibody and effector cells. The target cells can be in a monolayer on the substrate. The target cells can be plated at a density of between 2K and 100K per well (e.g., between 15K and 25K per well, or about 20K per well). The ratio of effector cells to target cells (E:T) can be 25:1. The E:T can be greater than 10:1, greater than 50:1, or greater than 100:1.

The antibody can be added at a concentration of between about 1 and about 100 μg/ml, between about 1 and about 50 μg/ml, or between about 2 and about 8 μg/ml. The can include adding to the assay medium two or more antibodies that bind to the target cells, three or more antibodies that bind to the target cells, or four or more antibodies that bind to the target cells. The antibody can be derived from a patient with an autoimmune disorder.

The target cells can express apical antigens. The method can include a preliminary step of screening the target cells for apical antigen expression. The target cells can be cancer cells or virally-infected cells. The cancer cells can be from a cell line. The cancer cells can be SKBR3 cells or MG63 cells.

The effector cells can comprise peripheral blood mononuclear cells (PBMCs), natural killer (NK) cells, monocytes, cytotoxic T cells or neutrophils. 30. Step (b) of the method can comprise adding to the target cells whole blood that has been partially enriched for the effector cells. Step (b) of the method can comprise adding whole blood to the effector cells, wherein the whole blood comprises the effector cells.

In another aspect, the present application features a method of screening a candidate antibody for the ability to induce ADCC against target cells, comprising: (a) monitoring the impedance between electrodes on a non-conducting substrate that supports the growth of target cells in the assay medium; and (b) adding effector cells and the candidate antibody that binds to the target cells to the assay medium; wherein a decrease in the impedance between the electrodes on the substrate following addition of the effector cells and the antibody is indicative of the ability of the candidate antibody to effect ADCC function against the target cells, and wherein an increase or no change in the impedance between the electrodes on the substrate following addition of the effector cells and the antibody is indicative of the inability of the candidate antibody to effect ADCC function against the target cells.

In another aspect, the present application features a method of identifying a patient having a disease associated with target cells that is suitable for treatment with a candidate antibody, the method comprising: (a) monitoring the impedance between the electrodes on a non-conducting substrate that supports the growth of target cells associated with the disease; (b) adding PBMCs isolated from the patient and the candidate antibody to the target cells; and (c) determining whether a change in the impedance between the electrodes on the substrate occurs following addition of the PBMCs and the antibody, wherein a decrease in impedance between the electrodes is indicative of the patient's suitability for treatment with the antibody, and wherein an increase or no change in the impedance between the electrodes on the substrate following addition of the PBMCs and the antibody is indicative of the patient's lack of suitability for treatment with the antibody.

In still another aspect, the present application features a method of screening a candidate compound for the ability to modulate ADCC, comprising:

(a) monitoring the impedance between electrodes on a non-conducting substrate that supports the growth of target cells in the assay medium; (b) adding to the assay medium effector cells and an antibody, in the presence and absence of the candidate compound, wherein the antibody binds to the target cells; and (c) comparing any change in the impedance between the electrodes on the substrate following addition of the effector cells and the antibody in the presence of the candidate compound with any change in the impedance between the electrodes on the substrate following addition of the effector cells and the antibody in the absence of the candidate compound, wherein a change in the impedance in the presence of the candidate compound that is greater than any change in the impedance in the absence of the candidate compound is indicative that the candidate compound has the ability to modulate ADCC. The compound to be screened can modulate autoimmune-related ADCC.

The present application also features a method as described herein which is a high-throughput assay, wherein the non-conducting substrate comprises two or more microtiter wells, each well comprising at least two electrodes, and monitoring the impedance between the electrodes in each well.

In another aspect, the present application features a quality control assay for an antibody, comprising: (a) monitoring the impedance between electrodes on a non-conducting substrate that supports the growth of target cells in an assay medium; and (b) adding effector cells and the antibody to the assay medium, wherein the antibody binds to the target cells; wherein a decrease in the impedance between the electrodes on the substrate following addition of the effector cells and the antibody is indicative that the antibody is suitable to be released for use in ADCC induction, and wherein an increase or no change in the impedance between the electrodes on the substrate following addition of the effector cells and the antibody is indicative that the antibody is not suitable to be released for use in ADCC induction.

In yet another aspect, the present application features a quality control assay for an antibody, comprising: (a) monitoring the impedance between electrodes on a non-conducting substrate that supports the growth of target cells in an assay medium; (b) adding effector cells and the antibody to the assay medium, wherein the antibody binds to the target cells; and (c) comparing any change in the impedance between the electrodes on the substrate following addition of the effector cells and the antibody with any change in the impedance for a control sample following addition of the effector cells and a control antibody; wherein a decrease in the impedance following addition of the effector cells and the antibody that is greater than any decrease in the impedance for the control sample following addition of the effector cells and the control antibody is indicative that the antibody is suitable to be released for use in ADCC induction, and wherein lack of a decrease in the impedance following addition of the effector cells and the antibody that is greater than any decrease in the impedance for the control sample following addition of the effector cells and the control antibody is indicative that the antibody is not suitable to be released for use in ADCC induction. In the quality control assay, a decrease in the impedance following addition of the effector cells and the antibody that is at least 25% greater than the decrease in the impedance for the control sample following addition of the effector cells and the control antibody can be indicative that the antibody is suitable to be released for use in ADCC induction.

The present application also features a method of screening a candidate compound for use as a therapeutic against an autoimmune disease, comprising:

(a) monitoring the impedance between electrodes on a non-conducting substrate that supports the growth of target cells in an assay medium, wherein the target cells are healthy cells; (b) adding to the assay medium effector cells and an antibody, with and without the candidate compound, wherein the antibody binds to the target cells, and wherein the effector cells are PBMCs from a subject diagnosed with the autoimmune disease; and (c) comparing any change in the impedance in the presence of the candidate compound with any change in the impedance in the absence of the candidate compound, wherein a decrease in the impedance in the absence of the candidate compound that is greater than any decrease in the impedance in the presence of the candidate compound is indicative that the candidate compound is suitable as a therapeutic agent against the autoimmune disease, and wherein lack of a decrease in the impedance in the absence of the candidate compound that is greater than any decrease in the impedance in the presence of the candidate compound is indicative that the candidate compound is not suitable as a therapeutic agent against the autoimmune disease

In another aspect, the present application features a method of determining whether a candidate antibody is suitable for treating a subject having an autoimmune disease, comprising: (a) monitoring impedance between the electrodes on a non-conducting substrate that supports the growth of target cells associated with the autoimmune disease; and (b) adding the candidate antibody and PBMCs isolated from the subject to the target cells; and (c) determining whether a change in the impedance between the electrodes on the substrate occurs following addition of the PBMCs and the candidate antibody, wherein a decrease in impedance between the electrodes is indicative that the antibody is suitable for treating the subject, and wherein the lack of a decrease in impedance between the electrodes is indicative that the antibody is not suitable for treating the subject.

In still another aspect, the present application features a method for determining an optimal concentration of an antibody for inducing an ADCC response, comprising: (a) monitoring the impedance between electrodes on a non-conducting substrate that supports the growth of two or more samples of target cells in an assay medium; and (b) adding effector cells and an antibody to the two or more samples of target cells, wherein the antibody binds to the target cells, and wherein the antibody is added at different concentrations to the two or more samples of target cells; wherein a decrease in the impedance between the electrodes on the substrate following addition of the effector cells and the antibody is indicative of ADCC function having been effected in the assay medium, and wherein the concentration of antibody that results in the greatest decrease in impedance is determined to be the optimal concentration.

In another aspect, the present application features a method of determining whether an antibody binds to an apical antigen on a target cell, comprising: (a) monitoring the impedance between electrodes on a non-conducting substrate that supports the growth of target cells in an assay medium; and (b) adding effector cells and the antibody to the target cells; wherein a decrease in the impedance between the electrodes on the substrate following addition of the effector cells and the antibody is indicative that the antibody binds to an apical antigen on the target cells.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

## BRIEF DESCRIPTION OF DRAWINGS

FIG. 1 depicts a graph plotting Cell Index over time for SKBR3 cells that were plated at 2-fold serial dilutions of 40K, 20K, 10K, or 5K per well, as indicated.

FIG. 2 depicts a graph plotting Cell Index over time for plated SKBR3 cells that were treated after 20 hours with additional fresh medium ("cells alone"), or with Triton X-100 ("cells + Triton") to induce cell death.

FIG. 3 depicts a graph plotting Cell Index over time for plated SKBR3 cells that were treated after 20 hours with fresh medium ("Targets alone"), Herceptin ("Targets + Herceptin"), or IgG control antibodies ("Targets + IgG").

FIG. 4 depicts a graph plotting Cell Index over time for SKBR3 cells alone ("targets") or PBMC ("effectors").

FIG. 5 depicts a graph plotting Cell Index over time for SKBR3 cells alone, PBMC alone, or SKBR3 cells that were treated with PMBC after 20 hours.

FIG. 6 depicts a graph plotting Cell Index over time for SKBR3 cells alone, SKBR3 cells treated with PBMC at 20 hours, or SKBR3 cells treated with PBMC and Herceptin[®] after 20 hours.

FIG. 7 depicts a graph showing a time course of SKBR3 target cell (T) lysis by PBMC effectors (E) at a 50:1 or 12.5:1 E:T ratio, with and without Herceptin[®] mAB as indicated.

FIG. 8A depicts a graph plotting percent lysis determined using a method as described herein for SKBR3 target cells treated with PBMC alone, SKBR3 target cells treated with PBMC plus the indicated concentrations of Herceptin[®], or SKBR3 target cells treated with PBMC plus control IgG. FIG. 8B depicts a graph plotting percent lysis determined using a standard Calcein-AM assay for SKBR3 target cells treated with PBMC alone, SKBR3 target cells treated with PBMC plus the indicated concentrations of Herceptin®, or SKBR3 target cells treated with PBMC plus control IgG.

FIG. 9 depicts a graph plotting Cell Index over time for SKBR3 and MG63 cells.

FIG. 10A depicts a graph plotting Cell Index for MG63 cells treated at 18-20 hours with PBMC alone, PBMC with RX1 target-specific antibody, or PMBC with IgG control antibody. FIG. 10B depicts a graph plotting Cell Index for SKBR3 cells treated at 18-20 hours with PBMC alone, PBMC with Hercepti[®] target-specific antibody, or PMBC with IgG control antibody.

## DETAILED DESCRIPTION

The present application provides materials and methods for detecting ADCC by measuring impedance between electrodes located in the surface on which the cells are plated. Any suitable device for measuring impedance can be used. In some embodiments, ADCC can be detected using a device having a non-conductive substrate having a plurality of electrode arrays positioned thereon, the arrays being connected to an impedance analyzer. For example, ADCC can be measured using a device having electrode arrays positioned on a substrate in a lower chamber, the lower chamber being separated from an upper chamber by a membrane. Such a device also can be used to detect migration of cells from the upper chamber to the lower chamber, where adherence to the substrate in the lower chamber is detected by an increase in impedance between the electrodes in the arrays in the lower chamber. Such devices are disclosed, for example, in WO2004/010103 and WO2004/010102.

Such devices also can be used to assess cytotoxicity of a compound on cells therein. For example, a compound can be added to cells being grown on a substrate containing electrodes, and the effect of the compound on impedance between the electrodes can be monitored, wherein a decrease in impedance between the electrodes is indicative of cell death. See, for example, WO2005/77104 and WO2005/047482. See, also, U.S. Publication No. 2005/0112544, which discloses methods that include measuring impedance to assess cellular cytotoxicity induced by Tamoxifen.

None of the above references disclose using the devices disclosed therein to assess ADCC. Unlike conventional cytotoxicity assays, which simply involve the addition of a candidate compound to a target cell, ADCC assays involve the addition of (a) an antibody that will bind to the target cell, and (b) effector cells that will bind to the antibody. Impedance measurements would not be expected to provide an accurate indication of the number of target cells in an ADCC assay since the target cells, the effector cells, and the antibody would be expected to adhere to the substrate. Thus, a high impedance reading could be due to: i) target cells adhering to the substrate because the antibody and effector cells had not induced ADCC; or ii) effector cells and antibodies adhering to the substrate following killing of target cells by ADCC. Devices such as those disclosed above therefore would not be expected to be useful in an ADCC assay.

Surprisingly, however, the inventors have found that devices such as those described in WO2005/77104 and WO2005/047482 can be used in assays for ADCC. In particular, the inventors have found that effector cells do not significantly adhere to the electrode-containing substrate, and that with suitable numbers of target cells, effector cell to target cell (E:T) ratios, and antibody concentrations, changes in impedance between electrodes on the substrate can provide an accurate indication of target cell numbers.

Assaying ADCC by detecting changes in impedance between electrodes in a substrate on which the target cells are grown has a number of significant advantages over standard ADCC assays. Unlike most standard ADCC assays, in some embodiments the methods provided herein are label-free. In some embodiments, the methods do not involve labeling cells with a radioactive or other label, making the assays easier and safer to perform than assays requiring labeled cells. Further, as discussed above, in standard ADCC assays, adherent cells are detached from the surface on which they are growing so that the cells can be labeled and the ADCC assay is performed in suspension. Since the methods provided herein are label-free, there is no need to detach cells, thereby removing a potential source of error due to perturbation of the adherent cells. Instead, the ADCC assays are performed with the cells attached to the substrate.

In addition, unlike most standard ADCC assays, in some embodiments the methods provided herein allow the number of cells to be followed in real-time, by measuring impedance at various timepoints after addition of the antibody and effector cells. The methods provided herein thus allow the kinetics of the ADCC reaction to be followed so that the ADCC kinetics of different antibodies can be compared. In some embodiments the methods are more sensitive than standard ADCC assays, allowing minor differences in the ADCC kinetics of different antibodies to be detected. In addition, the simplicity of the methods provided herein means that they are much faster than standard ADCC assays, and have the potential to be optimized for high-throughput screening of ADCC activity.

The methods provided herein can be conducted using a device described in WO2004/010102, WO2004/010103, WO2005/077104 or WO2005/047482, or a device based on these designs. In some embodiments, the methods provided herein can be conducted using the RT-CES® system manufactured by ACEA Biosciences (San Diego, CA). The ACEA RT-CES® system comprises three components: an electronic sensor analyzer, a device station, and a 16 well microtiter plate. The reader will appreciate that minor workshop variants of this system will be within the ambit of the skilled addressee. For example, the device can have a 96 well microtiter plate or a 256 well microtiter plate, rather than a 16 well microtiter plate, to allow more assays to be conducted simultaneously.

Microelectrode sensor arrays can be fabricated on glass slides using lithographical microfabrication methods, and the electrode-containing slides can be assembled to plastic trays to form electrode-containing wells.

The device station can receive the microtiter plate (e.g., the 16 well, 256 well, or 96 well microtiter plate), and can be capable of electronically switching any one of the wells to the sensor analyzer for impedance measurement. In operation, devices with cells cultured in the wells can be connected to the device station and placed inside an incubator. Electrical cables can connect the device station to the sensor analyzer. Using the RT-CES® software control, the sensor analyzer can automatically select wells to be measured, and can continuously conduct impedance measurements. The impedance data from the analyzer can be transferred to a computer, analyzed, and processed by the integrated software.

The impedance measured between electrodes in an individual well typically depends on electrode geometry, ionic concentration in the well, and whether there are cells attached to the electrodes. In the absence of cells, electrode impedance is determined mainly by the ion environment at the electrode/solution interface and in the bulk solution. In the presence of cells, cells attached to the electrode sensor surfaces can alter the local ionic environment at the electrode/solution interface, leading to an increase in the impedance. The more cells there are on the electrodes, the larger the increase in cell-electrode impedance.

To quantify cells based on the measured cell-electrode impedance, a parameter termed Cell Index (CI) is derived, according to the formula:

$$CI = \max_{i=1,\ldots,N}\left(\frac{R_{cell}(f_i)}{R_b(f_i)} - 1\right)$$

where $R_b(f)$ is the frequency-dependent electrode resistance (a component of impedance) without cells, $R_{cell}(f)$ is the frequency-dependent electrode resistance with cells present, and $N$ is the number of the frequency points at which the impedance is measured.

Thus, Cell Index is a quantitative measure of the cells in an electrode-containing well. Under the same physiological conditions, more cells attached onto the electrodes leads to a larger $R_{cell}(f)$ value, leading to a larger Cell Index value.

The methods provided herein can include measuring impedance at regular intervals and deriving a Cell Index from these impedance measurements according to the formula given above, thus allowing the ADCC reaction to be followed. The intervals at which impedance measurements are taken can depend on how closely it is desired to follow the kinetics of the reaction. For example, short intervals may be preferable if it is desired to follow the ADCC kinetics of an antibody already known to induce ADCC, whereas longer intervals may be satisfactory if the primary aim of conducting the assay is to ascertain whether a given antibody can induce ADCC at all. The methods can include taking impedance measurements at time points such as, for example, every 30 minutes, every 15 minutes, every 10 minutes, every 9, 8, 7, 6, 5, 4, 3, or 2 minutes, every minute, or more frequently than every minute.

The methods described herein can further include plating target cells on the substrate prior to addition of effector cells and antibody. For example, target cells can be plated on the substrate and allowed to grow for a period of time prior to addition of effector cells and antibody. Plating of target cells prior to addition of antibody and effector cells can allow the target cells to attach to the substrate and grow, resulting in an increase in impedance that may facilitate detection of a decrease in impedance following addition of effector cells and antibody. The target cells typically should not be allowed to overgrow, however, as the target cells attached to the substrate may become inaccessible to the antibody due to cells growing above them. In such a case, it would not be possible to detect ADCC. It is well within the ability of the skilled person to conduct experiments to determine the optimum length of time that target cells should be plated before addition of effector cells and antibody. In some embodiments, target cells can be plated on the substrate 10-30 hours prior to addition of effector cells and antibody. For example, target cells can be plated on the substrate 18-24 hours (e.g., 19-21 hours) prior to addition of effector cells and antibody.

The density at which the target cells are plated can depend on the size of the cells and the rate at which they grow. The detection system typically has a maximal limit, above which it is unable to detect further growth of the cells. Target cells thus should be plated at a density below this limit to allow changes in impedance due to death of the cells to be detected. However, target cells also should be plated at a density that is high enough to allow the cells to have an effect on impedance during the initial period of growth, so that any decrease in impedance following addition of effector cells and antibody can be detected. In some embodiments, target cells can be plated at a density between 2K and 100K in a standard well of 19.6 mm$^2$ (e.g., a density between 10K and 60K in a standard well of 19.6mm$^2$, a density between 15K and 25K in a standard well of 19.6mm$^2$, or a density around 20K in a standard well of 19.6mm$^2$).

The length of time during which impedance measurements are made, i.e., the length of the assay, can vary depending on the period between plating of the target cells and addition of effector cells and antibody. A reduction in impedance due to ADCC generally can be detected within a couple of hours after addition of antibody and effector cells. In some cases, however, it may be desirable to continue monitoring impedance for a period after onset of cell killing by ADCC, to determine the extent of cell death and to ascertain whether and when any cell regrowth occurs. The methods provided herein thus may include taking impedance measurements for, without limitation, 24 hours, 36 hours, 48 hours, 72 hours, 100 hours, 200 hours, or more than 200 hours after plating of the target cells.

The methods provided herein can employ any number of effector cells and any number of target cells, provided, as discussed above, that the number of target cells is not so high as to prevent accurate detection of ADCC. The methods can employ more effector cells than target cells. The ratio of effector cells to target cells (E:T) can be, for example, greater than 10:1, greater than 20:1, greater than 50:1, greater than 100:1, or around 25:1.

The antibody can be added to the target cells at any concentration. For example, the antibody can be added to the plated target cells at a concentration of, for example, between about 1 and about 1000 μg/ml between about 1 and about 500 μg/ml, between about 1 and about 100 μg/ml, between about 1 and about 75 μg/ml, between about 1 and about 50 μg/ml, between about 1 and about 25 μg/ml, between about 1 and about 10 μg/ml, between about 2 and about 8 μg/ml, or between about 4 and about 6 μg/ml.

In some embodiments, the methods described herein can be used to determine the optimal concentration of an antibody that induces an ADCC response by comparing the ADCC response obtained using different antibody concentrations. For example, a method can include (a) monitoring the impedance between electrodes on a non-conducting substrate that supports the growth of two or more (e.g., two, three, four, five, or more than five) samples of target cells in an assay medium; and (b) adding effector cells and an antibody to the two or more samples of target cells, wherein the antibody binds to the target cells, and wherein the antibody is added at different concentrations to the two or more samples of target cells; wherein a decrease in the impedance between the electrodes on the substrate following addition of the effector cells and the antibody is indicative of ADCC function having been effected in the assay medium, and wherein the concentration of antibody that results in the greatest decrease in impedance is determined to be the optimal concentration.

Effector cells and antibody can be added to the target cell medium at the same time or separately. For example, effector cells can be added to the medium before antibody, or antibody can be added before effector cells. The methods described herein can employ any combination of target cells, effector cells, and antibodies, and the selection of target cells, effector cells, and antibodies used in the methods can depend on the purpose of the assay. Examples of suitable target cells, effector cells and antibodies for use in the methods provided herein are discussed below, as are possible applications of assays employing these target cells, effector cells and antibodies.

In some embodiments, the target cells are adherent cells. In some embodiments the target cells express apical antigens. Many cells are polarized, and express different antigens on their apical and basolateral surfaces (Nelson et al. (2003) Nature 422:766-774). For example, epithelial cells express different apical and basolateral antigens. When a standard ADCC assay is conducted in suspension, the antibody may bind to both apical and basolateral antigens. In the methods provided herein, however, where the cells adhere to the substrate, the antibody may only bind to apical antigens. The ability of an antibody to induce ADCC thus demonstrates that the antibody binds to an apical antigen on the target cells. The methods provided herein thus can be used to determine whether a particular antibody binds to an apical antigen on the target cells. For example, a method can include: (a) monitoring the impedance between electrodes on a non-conducting substrate that supports the growth of target cells in an assay medium; and (b) adding effector cells and the antibody to the target cells; wherein a decrease in the impedance between the electrodes on the substrate following addition of the effector cells and the antibody is indicative that the antibody binds to an apical antigen on the target cells. Some therapeutic antibodies are only able to access apical antigens *in vivo.* For example, some antibodies are only able to access and bind apical antigens expressed on the surface of tumors. The ability of an antibody to induce ADCC by binding to apical antigens may thus be an indicator of the therapeutic effectiveness of the antibody *in vivo.*

The methods provided herein can include a preliminary step of screening target cells for expression of apical antigens. Such a preliminary step can employ, for example, RT-PCR or FACS to identify target cells expressing apical antigens that may be suitable for use in the methods provided herein.

Any suitable target cell can be used. Examples of cells that express apical antigens include, without limitation, tumor cells and epithelial cells such as those known in the art. For example, mucin glycoproteins such as DF3 antigen and MUC1 are expressed on the apical surface of epithelial cells and are over-expressed on the apical surface of epithelial tumors (Snijdewint et al. (2001) Int. J. Cancer 93:97-106; and Hayes et al. (1990) J. Immunol. 145:962-970). In addition, a variety of tumor cells express a cell surface receptor with high affinity for folic acid on their apical surfaces (Lu et al. (2002) Cancer Immunol. Immunother. 51:153-162).

The target cells can be diseased cells such as, for example, cancer cells or virally-infected cells. These target cells may be cell lines obtained from cell line banks. Alternatively, the cells can be obtained from an individual having a disease or a disorder. For example, target cells can be obtained from a tumor biopsy of a cancer patient.

Cancer cells that can be used as target cells include, but are not limited to, cells associated with Hodgkin's Disease, non-Hodgkin's B-cell lymphomas, T-cell lymphomas, malignant lymphoma, lymphosarcoma leukemia, chronic lymphocytic leukemia, multiple myeloma, chronic myeloid leukemia, chronic myelomonocytic leukemia, myelodysplastic syndromes, myeloproliferative disorders, hypereosinophilic syndrome, eosinophilic leukemia, multiple myeloma, X-linked lymphoproliferative disorders, esophageal cancer, stomach cancer, colon cancer, colorectal cancer, pancreatic cancer and gallbladder cancer, cancer of the adrenal cortex, ACTH-producing tumor, bladder cancer, brain cancer (e.g., neuroblastomas and gliomas), Ewing's sarcoma, head and neck cancer (e.g., mouth cancer and larynx cancer), kidney cancer (e.g., renal cell carcinoma), liver cancer, lung cancer (e.g., small and non-small cell lung cancers), malignant peritoneal effusion, malignant pleural effusion, skin cancers (e.g., malignant melanoma, tumor progression of human skin keratinocytes, epithelial cell carcinoma, squamous cell carcinoma, basal cell carcinoma), mesothelioma, Kaposi's sarcoma, bone cancer (e.g., osteomas and sarcomas such as fibrosarcoma and osteosarcoma), cancers of the female reproductive tract (e.g., uterine cancer, endometrial cancer, ovarian cancer, and cervical cancer), breast cancer, prostate cancer, retinoblastoma, testicular cancer, and thyroid cancer.

Cancer cell lines that can be used as target cells can be any immortalized adherent cell lines obtained, for example, from a cell bank. Examples of suitable adherent immortalized cell lines include, but are not limited to: SKBR3, MG63, CCD-1070Sk, hTERT-HME1 [ ME16C ], BJ, ATRFLOX [ Mutatect ], KEL FIB, HT-1197, LL 97A (AlMy), NCI-H510A [ H510A; NCI-H510 ], HT-29, SW756, 293, IMR-90 [IMR90], HIAE-55 [part of the Wistar Special Collection], SW1417 [SW-1417], Hs 737.T, NCI-H661 [H661], CCD 841 CoN, Hs 792(C).M, Per Sel, NCI-H810 [H810], Panc 05.04, ZR-75-30, T-47D, WISH, HBE135-E6E7, ARPE-19/HPV-16, 10.014 pRSV-T, GH354, MDA-MB-436, T98G [T98-G],

EP 2 246 700 A1

Calu-6, BEAS-2B, G-292, clone A141B1, Detroit 539, MNNG/HOS Cl #5 [R-1059-D], BT-549, NCI-H1915 [H1915], Hs 181.Sk, Hs 839.T, RD, SK-N-MC, 20B8, NCI-H292 [H292], Hs 863.T, Hs 181.Tes, Malme-3M, Hs 617.Mg, JAR, Hs 144.We, Hs 742.Sk, Hs 875.T, TE 161.T, Hs 738.St/Int, Hs 895.T, RWPE-1, TE 130.T, TE 84.T, HCC1008, Hs 894 (E).Lu, SK-LMS-1, HFF-1, MRC-5 [MRC5], IRR-MRC-5 [ATCC X-55; ATCC X55; irradiated MRC-5], WI-38 [WI 38], HeLa, HEK001, FHs 74 Int, Detroit 548, Detroit 573, SW-13, 293T/17, C 211, Amdur II, IMR-32, HeLa [Chang Liver], CHP 3 (M.W.), CHP 4, LL 47 (MaDo), HEL 299, Detroit 562, CCD-11Lu, KB, A549 [A-549], MDA-MB-134-VI, HLF-a, TE 354.T, HeLa 229, HeLa S3, COLO 320DM, clone 1-5c-4 [Wong-Kilbourne derivative (D) of Chang conjunctiva], CCD-13Lu, CCD-8Lu, CCD-19Lu, CCD-16Lu, AV3, Hs888Lu, CCD-25Lu, COLO 320HSR [COLO 320 HSR], DLD-1, COLO 205, COLO 201, HCT-15, SW837 [SW-837], LoVo, SW48 [SW-48], SW1463 [SW 1463; SW-1463], SW 1353 [SW 1353; SW-1353], 1205Lu [part of the Wistar Special Collection], HCT-8 [HRT-18], AGS, HCT 116, T84, SNU-C2B, SNU-C2A, NCI-H716 [H716], NCI-H747 [H747], NCI-H498 [H498], LS123, NCI-H1688 [H16881, CFPAC-1, L-I32, TE 353.Sk, intestine 407, FL, Detroit 525, Detroit 510, C32, WI-38 VA-13 subline 2RA [part of the Wistar Special Collection], citrullinemia, Cri du Chat, WI-26 VA4 [part of the Wistar Special Collection], BeWo, WM35 [part of the Wistar Special Collection], MSTO-211H, WRL 68, NCI-H295 [H295], MCF 10A, MCF 10F, RWPE2-W99, SV-HUC-1, HCC202, C3A [HepG2/C3A; derivative of Hep G2 (ATCC HB-8065)], MCF-10-2A, 293 c18, F.thy 62891, Lei Cap, Sal Mat, HCE-2 [50.B1], A2058, RWPE-2, Am Ric, Lo Ren, Ron Har, H69AR, hFOB 1.19, Mar Vin, SCC-4, Be Sal, Hs27, B-3, Lu Vin, Ma San, El Don, SK-N-AS, NCI-H1734 [H-1734; H1734 ], LNZTA3WT4 [LNZTA3p53WT4; WT4 ], LNZTA3WT11 [LNZTA3p53WT11; WT11], Lo Wen, NCI-H2227 [H2227], COLO 829, HKB-11, Ce Ar, 90.74, HRT-18G, Be Ar, Em Ar, Win Mec, Ce Geg, TOV-21G, TOV-112D, OV-90, Ja Bos, Fe Bos, La Bel, Bo Gin, HS-5, Le Ana, Mel Neg, La Bel II, HEP G2/2.2.1, ProPakA. 6 [PPA.6 ; ProPak-A.6], LNCaP clone FGC [LNCaP.FGC], HCN-1A, HX [HT1080 xeno], HP [HT1080 poly], 2A, Ne Loc, Jay Sen, Bi Fin, and Ray Hot.

Virally-infected cells that can be used as target cells include, for example, cells infected with Epstein Barr Virus, HIV, influenza virus, polio virus, hepatitis A virus, Hepatitis B virus, Hepatitis C virus, Varicella zoster virus, Rubella virus, measles virus, Herpes Simplex Virus, Dengue virus, papilloma virus, respiratory syncytial virus, or rabies virus.

The target cells used in the methods provided herein also can be healthy cells. ADCC may be involved in the killing of healthy cells in patients with autoimmune diseases such as autoimmune thyroid disorders, myasthenia gravis, rheumatoid arthritis, systemic lupus erythematosus, immune haemolytic anaemia (penicillin induced), autoimmune hepatitis (AIH), Graves' ophthalmopathy, HIV [CD4 depletion], autoimmune enteropathy, coeliac disease, myasthenia gravis (MG), Behcet's disease, thyroid-associated ophthalmopathy, autoimmune thyroiditis, viral myocarditis, autoimmune heart disease, inflammatory bowel disease, ulcerative colitis, Chagas disease, vitiligo, Crohn's disease, idiopathic thrombocytopenic purpura, autoimmune neutropenia, childhood epilepsy, Kawasaki disease, autoimmune chronic active hepatitis, diabetes mellitus, multiple sclerosis, anti-glomerular basement membrane (GBM) nephritis, chronic active liver disease (CALD), glomerulonephritis (from immune complexes), thrombotic thrombocytopenic purpura (TTP), unexplained male infertility, and transplant rejection. The use of healthy cells as target cells in the methods provided herein thus can be useful in research into how antibodies and effector cells kill healthy cells by ADCC in autoimmune disease or transplant rejection. Where the target cells are healthy cells, they can be, for example, cell lines obtained from cell banks or cells obtained from the tissue of an individual having an autoimmune disease.

In some cases, a combination of more than one type of target cell can be used to, for example, more closely replicate the situation *in vivo* where the antibody may target organs and tissues comprising several cell types. The target cells thus can include more than one cell line or can include cells from more than one tissue. In some embodiments, the target cells include two or more or three or more cell types.

The methods described herein can be used to screen any antibody for the ability to induce an ADCC response. The present application thus provides methods for screening a candidate antibody for the ability to induce ADCC against target cells. The methods comprise (a) monitoring the impedance between electrodes on a non-cnnducting substrate that supports the growth of target cells in an assay medium; and (b) adding to the assay medium effector cells and the candidate antibody (e.g., an antibody that binds to the target cells); wherein a decrease in the impedance between the electrodes on the substrate following addition of the effector cells and the antibody indicates the ability of the candidate antibody to effect ADCC against the target cells.

As used herein, "a decrease in impedance" refers to any reduction in impedance between the electrodes on a substrate having target cells plated thereon. A decrease in impedance can be, for example, a reduction of about 1% or more, about 5% or more, about 10% or more, about 15% or more, about 20% or more, about 25% or more, about 40% or more, about 50% or more, about 60% or more, about 75% or more, about 80% or more, about 90% or more, or about 100% in impedance as compared to a previously determined impedance.

The antibody or candidate antibody used in the methods provided herein can be, for example, a monoclonal antibody, a chimeric antibody, a humanized antibody, or a human antibody. Antibodies that can be used in the methods described herein also include antibodies that have been identified as having therapeutic potential (e.g., antibodies that have already undergone clinical trials).

As used herein, the term "antibody" refers to intact molecules as well as to fragments thereof, such as Fab, F(ab')2 and Fv, which are capable of binding to the target cells. By "binding to target cells" is meant that an antibody is immunospecific for an antigen of the target cells, e.g., an apical antigen on the target cells.

The term "immunospecific" means that the antibody has substantially greater affinity for the antigen on the target cell than affinity for other proteins (e.g., other related proteins).

By "substantially greater affinity" is meant that an antibody has a measurably higher affinity for an antigen on a target cell as compared with affinity for known immunoglobulin domain-containing cell surface recognition molecules. For example, the affinity can be at least 1.5-fold, 2-fold, 5-fold, 10-fold, 100-fold, $10^3$-fold, $10^4$-fold, $10^5$-fold, or $10^6$-fold greater for an antigen on a target cell than for known immunoglobulin domain-containing cell surface recognition molecules.

The methods provided herein also can be used as release or stability assays for quality control (QC) purposes. For example, methods as described herein can be used to test antibody production lots, wherein the presence of ADCC activity or the level of ADCC activity is used to certify the antibody product as meeting quality control guidelines (e.g., in GMP (Good Manufacturing Practice) or other standards). In some cases, the mere presence of ADCC activity can be used as a qualitative determination that an antibody meets quality control guidelines and is suitable for release. Such methods thus can require no interpretation beyond a determination that impedance is decreased in the presence of the antibody being tested. In some embodiments, a sample of the lot of an ADCC antibody to be certified can be deliberately degraded and used as a negative control for comparison with the antibody lot to be released. The real time ADCC readout curve for the two samples can be compared, and a quantitative or qualitative cut-off can be established for quality control certification. The exact concentration-dependent response and assay parameters can be determined by trial and error.

Similarly, methods described herein can be used to certify small molecule or other inhibitors of ADCC activity for QC and stability purposes, by monitoring the decrease in real time ADCC signal in response to their addition to target cells.

Examples of available antibodies that may act via ADCC include, without limitation, antibodies for treatment of allergy and asthma, such as Daclizumab (Protein Design Labs/Roche) and CAT-354 (Cambridge Antibody Technology); antibodies for treatment of autoimmune disorders, such as MDX-H210 (Medarex/Novartis), Campath* alemtuzumab (ILEX Oncology), and Daclizumab (Protein Design Labs/Roche); antibodies for treatment of cancer, such as Campath® (ILEX Oncology), R1550 (huHMFG1, Therex, Antisome/Roche), Herceptin® (Genentech), Omnitarg (Genentech), Tastuzumab-DMI1 (Genentech/Immunogen), MDX-010 (Medarex), Avastin® (Genentech), Mab-17-I (edrecolomab-IGN101, Igeneon, EDR or Panorex- GSK), labetuzumab, (IMMU 105) (Immunomedics), Tarvacin (Peregrine), Theragyn, Therevex (Pemtumomab) (Antisoma/Roche), TheraCIM hR3 (YM Biosciences/Oncoscience), HuMax-EGFr(Genmab), SGN-40 (Seattle Genetics), SGN-30 (Seattle Genetics), Rituxan® (Rituzan® Genentech/BiogenIdec), HuMax-CD4 (Genmab), MDX-060 (Medarex), Siplizumab (MedImmune), AME-133 (Applied Molecular Evolution/Lilly), galiximab (Anti-CD80 Mab BiogenIdec), HuMax-DC20 (HuMax cd20) (Genmab), LymphoCide™ (epratuzumab, Immunomedics), MDX-010+gp100 peptide (Medarex), MDX-010 +/- chemotherapy (Medarex), MDX-010 + melanoma peptides (Medaex), AHM (Roche/Chugai), OvaRex (AltaRex/Unither Pharma-ceuticals), J59I (Biovation/Millennium), Rencarex (WX-G250) (Wilex/Centocor/Johnson & Johnson), WX-G250+IL-2/IFN (Wilex), TRAIL-R1mAb (HGS-ETR1Cambridge Antibody Technology/Human Genome Sciences), TRAIL-R2mAb (HGS-ETR2Cambridge Antibody Technology/Human Genome Sciences), Vitaxin (MEDI-522 MedImmune), WX-K931 (Wilex), MT201 (Micromet/Serano), MDX-214 (Medarex), Erbitux (cetuximab, IMC-C255 Imclone, Bristol-Myers Squibb), MEDI-507 (Medimmune), TRU-015 (an antibody derivative, Trubion), Matuzumab (EMD-72000, EMD Pharmaceuticals/ Merck KGaA), Mab ICR62, CHIR-12.12 (Chiron/XOMA), huG1-M195 (NCI) MOR102 (MorphoSys), Remitogen (1D10, anti-MHC class II, Protein Design Labs), and IGN312 (Igeneon); antibodies for treatment of cardiovascular diseases, such as ABC-48 (AERES); antibodies for treatment of infectious diseases, such as MDX-010 (Medarex); antibodies for treatment of inflammatory diseases, such as Humira™ (Cambridge Antibody Technology/Abbott), Anti-CD11a MAb (Biovation), MLN02 (Millennium/Genentech/Roche), Daclizumab (Protein Design Labs), Enbrel (fusion of TNF-receptor and IgG1 (Amgen/Wyeth), Remicade (Centocor), and TRU-016 (an antibody derivative, Trubion); antibodies for treatment of kidney diseases, such as Humira™ (Cambridge Antibody Technology/Abbott) and Rituxan® (Genentech/BiogenIdec); and other antibodies such as AMG162 (Amgen) for osteoporosis, Erbitux - C255, BTI-322, Etanercept, and Infliximab.

Where the methods provided herein are used to investigate ADCC responses in autoimmune diseases, the antibody can be, for example, an auto-antibody obtained from an individual having an autoimmune disease. Such antibodies can be obtained using, for example, methods known to those skilled in the art (e.g., affinity purification).

In some cases, it may be desirable to add more than one antibody to the assay medium in order to determine, for example, if the antibodies act synergistically or whether they interfere with one another in terms of the ability to induce ADCC. The methods provided herein thus can include adding two, three, four, five, or more than five antibodies to the assay medium.

The effector cells used in the methods provided herein typically are cells that express one or more Fcγ receptors. Suitable effector cells include, but are not limited to, peripheral blood mononuclear cells (PBMCs), natural killer (NK) cells, monocytes, cytotoxic T cells, and neutrophils.

Several families of Fcγ receptors exist, and different effector cells express different Fc receptors. For example, neutrophils commonly express FcγRI (CD64), FcγRII (CD32) and the lipid anchored isoform of FcγRIII (CD16), whereas natural killer (NK) cells express only the transmembrane isoform of FcγRIII. Furthermore, polymorphisms exist within the Fc receptors. For example, the FcγRIII on NK cells contains a Phe/Val polymorphism at position 158 that has been shown to influence IgG binding. Effector cells used in the methods described herein can be selected on the basis of the Fc receptor that they express and, in some embodiments, the polymorphic form of the Fc receptor they express. The methods described herein thus can be used to determine whether a particular combination of an antibody and an effector cell expressing a known Fcγ receptor (e.g., with a particular polymorphism) is capable of killing a target cell by ADCC. Such studies may be useful in general research into the mechanism of IgG binding by Fc receptors, and may allow identification of residues in both the Fc receptors and the IgG Fc region that are crucial for binding. In turn, such studies may enable development of therapeutic antibodies having Fc region sequences that display optimal binding with Fc receptors on effector cells, thus maximizing ADCC. Such studies also may be used to determine the optimal effector cell genotype for an existing therapeutic antibody to be able to induce an ADCC response, so that a prospective patient can be tested for the presence of Fc receptors with the optimal genotype.

In some embodiments, the effector cells used in the methods described herein are PBMCs. PBMCs are a mixture of monocytes and lymphocytes that can be isolated from whole blood using, for example, standard experimental protocols described in the art and in the Examples below. The variety of cells found in PBMCs would be expected to result in confusion in the impedance readings due to non-specific adherence to the substrate. Surprisingly, however, the inventors have found that a change in impedance or Cell Index following addition of an antibody and PBMCs is an accurate indication of ADCC of target cells. Whole blood, or blood that has been at least partially purified such that it is enriched, or partially enriched, for effector cells (e.g., PBMCs), also may be useful in the methods provided herein.

The methods provided herein have a wide variety of clinical applications. As discussed above, polymorphisms exist within human Fc receptors that have an effect on the ability of effector cells to bind an antibody, and that thus may affect the ability of the effector cells to mediate ADCC. The ability of effector cells in an individual to bind a therapeutic antibody can have a major impact on the clinical efficacy of the antibody. For example, the anti-CD20 IgG antibody Rituxa® used in the treatment of B lymphoproliferative malignancies can generate a better clinical outcome in patients homozygous for the FcγRIII158V genotype than in patients homozygous for the FcγRIII158F genotype, possibly due to the fact that NK cells bearing FcγRIII158V bind more effectively to the Fc region of Rituxan®.

The ability to use PBMCs from individuals (e.g., patients) as effector cells means that the methods provided herein can be used to determine whether a particular therapeutic antibody is suitable for treatment of a particular patient having a disease or disorder associated with target cells. Accordingly, in some embodiments the effector cells can be PBMCs obtained from a patient having a disease associated with the target cells, and the antibody can be a candidate therapeutic antibody for treating the patient. This document thus provides methods of identifying a subject as having a disease associated with target cells that is suitable for treatment with a candidate antibody. The methods can comprise (a) monitoring impedance between the electrodes on a non-conducting substrate that supports the growth of target cells associated with the disease; (b) adding PBMCs isolated from the patient and the candidate antibody to the target cells; and (c) determining whether a change in the impedance between the electrodes on the substrate occurs following addition of the PBMCs and the antibody, wherein a decrease in impedance between the electrodes is indicative of ADCC and thus of the patient's suitability for treatment with the antibody.

In some embodiments, the target cells can be cells associated with B lymphoproliferative malignancies, and the candidate antibody can be Rituxan®. According to such embodiments, methods as provided herein can be used to determine whether a patient having a B lymphoproliferative malignancy is suitable for treatment with Rituxan®. The method may, of course, be conducted with PBMCs from a particular patient in combination with any of the other antibodies and target cells described herein to identify the optimal antibody for treatment of the patient. Further, when PBMCs are identified as capable of inducing ADCC in combination with a particular antibody, the genotype of the PBMCs may be determined and the PBMCs of other individuals may be tested using standard genotyping methods to establish whether they are suitable for treatment with that antibody.

Where PBMCs from an individual are used as the effector cells, the target cells also may be cells from the individual (e.g., cancer cells obtained from a biopsy), so that the method is a personalized method of determining whether a particular candidate antibody in combination with the subject's PBMCs is capable of killing target cells in the patient by ADCC. As more antibody products become available, the methods provided herein will allow selection of the best antibody product to optimize clinical results in a particular patient.

The methods provided herein also can be adapted to screen compounds for the ability to increase or decrease ADCC induced by different combinations of effector cells and antibodies. Accordingly, this document provides methods of screening compounds for the ability to modulate ADCC. The methods can comprise (a) monitoring the impedance between electrodes on a non-conducting substrate that supports the growth of target cells in an assay medium; (b) adding the compound, effector cells, and an antibody that binds to the target cells to the assay medium; and (c) determining whether the compound modulates ADCC by comparing any change in the impedance between the electrodes on the substrate following addition of the effector cells and the antibody in the presence of the compound with any change in the impedance between the electrodes on the substrate following addition of the effector cells and the antibody in the absence of the compound.

As discussed herein, a decrease in impedance following addition of effector cells and antibody is indicative of ADCC having been effected in the assay medium. A further decrease in impedance observed in the presence of a compound being screened may be an indication that the compound increases ADCC. Conversely, an increase in impedance in the presence of the compound may be an indication that the compound decreases ADCC. In some situations, it may be desirable to identify compounds that increase ADCC. For example, the methods may be used to identify compounds that increase the ADCC response induced by a therapeutic antibody and PBMCs against cancer cells. In other situations, it may be desirable to identify compounds that decrease ADCC. For example, the methods may be used to identify compounds that decrease the ADCC response induced by antibodies and PBMCs from a patient with an autoimmune disease against healthy target cells. The finding that a compound does not act as a modulator of ADCC also may be useful in some situations, since it shows that the compound (which may itself have another therapeutic purpose) does not interfere with the ability of a particular effector cell-antibody combination to induce ADCC.

Such methods can employ any of the target cells, effector cells, and antibodies described herein. In some embodiments, the effector cell-antibody combination employed can be a combination that has already been identified as being capable of inducing an ADCC response against the target cells in question. The compound to be screened can be added to the target cells at the same as the effector cells and the antibody. Alternatively, the compound to be screened can be added to the target cells before the effector cells and the antibody, or after the effector cells and the antibody.

Compounds that can be screened for the ability to modulate ADCC in the methods provided herein include, but are not restricted to, peptides, peptoids, proteins, lipids, metals, small organic molecules, RNA aptamers, antibiotics and other known pharmaceuticals, polyamines, and combinations or derivatives thereof. Small organic molecules typically have a molecular weight from about 50 daltons to about 2,500 daltons (c.g., from about 300 to about 800 daitons). Candidate compounds can be derived from large libraries of synthetic or natural compounds. For example, synthetic compound libraries are commercially available from MayBridge Chemical Co. (Revillet, Cornwall, UK) or Aldrich (Milwaukee, WI). Alternatively, libraries of natural compounds in the form of bacterial, fungal, plant and animal extracts may be used. Additionally, candidate compounds may be synthetically produced using combinatorial chemistry either as individual compounds or as mixtures.

In some embodiments, the compound to be screened can be a compound that is already used in treatment of a disease with which the target cells are associated, and the antibody may be an antibody developed for the treatment of that disease. For example, where the target cells are cancer cells, the compound to be screened can be a chemotherapeutic agent and the antibody can be a therapeutic antibody developed for the treatment of cancer. Methods as provided herein thus can be used to determine whether the chemotherapeutic agent increases or decreases killing of the target cells by the antibody by ADCC. Similarly, the compound to be screened can be one that modulates ADCC in a patient with an autoimmune disorder. In such cases, the antibody to be added can be an antibody that causes autoimmune activity via ADCC in an individual with an autoimmune disease.

In some embodiments, the methods described herein can be high-throughput methods that simultaneously assess the ability of multiple combinations of antibodies, effector cells, and target cells to produce an ADCC response, optionally in the presence of compounds that may modulate the ADCC response. In such embodiments, the target cells can be plated on a non-conducting substrate containing two or more wells, each well comprising at least two electrodes, and the method can include monitoring any change in impedance between the electrodes in each individual well. The RT-CES® system and associated software described herein is adapted for use in high-throughput assays.

In some embodiments the target cells, antibodies, and effector cells added to each well may be the same or different depending on the purpose of the assay. In some embodiments, for example, the method can be used to simultaneously test the ability of multiple candidate therapeutic antibodies to induce ADCC against a variety of target cells in the presence of PBMCs from one patient or PBMCs from several patients. Such high-throughput methods also can be used to simultaneously test the ability of a single candidate antibody to induce ADCC against the same target cell, or a variety of different target cells, in the presence of the same effector cells at a variety of antibody concentrations. In addition, such high-throughput methods can be used to simultaneously compare the ADCC kinetics of a variety of different antibodies. High-throughput assays also can be used to screen compounds for the ability to modulate ADCC responses, as discussed above.

Further, use of a method as described herein as part of a high-throughput method allows control experiments to be conducted in parallel to the method for assaying ADCC activity described above. For example, a suitable control assay can include plating and growing target cells and monitoring impedance in the absence of effector cells and antibody. Further control experiments may comprise adding effector cells alone or adding antibody alone to the plated target cells.

The invention will be further described in the following example, which does not limit the scope of the invention described in the claims.

**EXAMPLE**

*1. Introduction*

The ACEA Biosciences (San Diego, CA) RT-CES® (Real Time Cell Electronic Sensor) system utilizes an electronic readout of impedance to non-invasively quantify cellular status in real-time. Cells are seeded in E-Plate micro-titer plates (ACEA Biosciences), which are integrated with microelectronic sensor arrays. The interaction of cells with the microelectrode surface leads to generation of a cell-electrode impedance response, which indicates the status of the cells in terms of morphology, quality of adhesion and number.

The ACEA system was used to develop a real-time ADCC assay for adherent cells. Two cell lines were used: SKBR3 (a mammary gland adenocarcinoma cell line); and MG63 (an osteoblastic cell line). SKBR3 is an adherent cell line that overexpresses the HER-2 antigen. Herceptin®, a humanized antibody against HER-2, mediates killing of SKBR3 cells by ADCC in the presence of effector cells. MG63 is an adherent cell line that overexpresses M-SCF. Chir-RX1, a humanized IgG1 antibody against M-CSF, mediates ADCC against MG63 cells in the presence of effector cells.

Experiments were conducted to determine whether the ACEA system could be used to monitor killing in real-time of SKBR3 and MG63 target cells by ADCC mediated by PBMC effector cells and either Herceptin® or RX-1.

*2. Target cell, antibody, and PBMCpreparation*

SKBR3 cells (ATCC, Manassas, VA; catalog #HTB-30) were cultured in DME-15 Medium.

Humanized antibody Herceptin® (Genentech Corporation, South San Francisco, CA; trade name Trastuzumab) was reconstituted with water to make a 21 mg/ml stock solution before use. Herceptin® is stable for 30 days after reconstitution.

Human PBMC was prepared fresh from human blood obtained from healthy volunteer donors. Human venous blood samples (8.5 ml) were collected in yellow-top ammonium citrate (ACD-A) tubes. The tubes were inverted 10-15 times, and whole blood was removed from each yellow-top tube and transferred into one 50 ml conical tube. Blood was diluted 1:3 with PBS in 2% FBS. Twelve ml of Ficoll-Hypaque (Amersham Biosciences, Piscataway, NJ; cat #17-1440-02) was dispensed slowly underneath blood/PBS mixture. Samples were centrifuged at room temperature at 2350 rpm for 30 minutes before removing the upper layer (plasma/PBS) by aspiration. Buffy coats were collected with sterile pipettes and pooled into a 50 ml conical tube. If visible clumps of WBC remained below the buffy coat, all of the WBC material was collected, with care not to remove excess Ficoll-Hypaque. Sterile PBS-2% FBS was added to bring the volume to 30 ml, and the mixture was mixed by inversion. The diluted PBMC suspensions was centrifuged at 250 x g (1046 rpm for Beckman GH3.8 rotor) at room temp for 17 minutes, and the cell pellet was collected. The PBMC pellet was suspended in 2.5 ml R-2 Medium (RPMI-1640 medium with 2% heat-inactivated FBS), mixed thoroughly, and transferred to a 15 ml conical tube.

PBMC were washed once with R-2 medium, and the number of viable cells was checked by Trypan blue (Invitrogen cat. #15250-061 or equivalent) exclusion.

*3. Assay of ADCC killing of SKBR3 cells by Herceptin® and PBMC*

Prior to conducting the ADCC assay, several experiments were conducted to assess the effect of the SKBR3 cells alone, with the Herceptin® antibody, or with PBMC effector cells, on the Cell Index readout from the RT-CES® system.

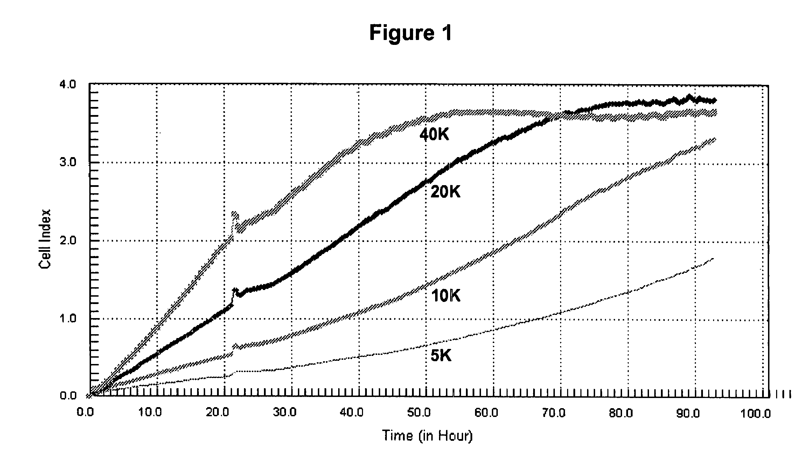

SKBR3 cells were plated at 40K, 20K, 10K, or 5K per well and placed in the RT-CES® reader. Fresh medium was added after 20 hours and Cell Indices were monitored for 100 hours. As shown in Figure 1, the Cell Index increased as the SKBR3 cells proliferated. The Cell Index had a maximal detection limit of 60K to 160K cells per well.

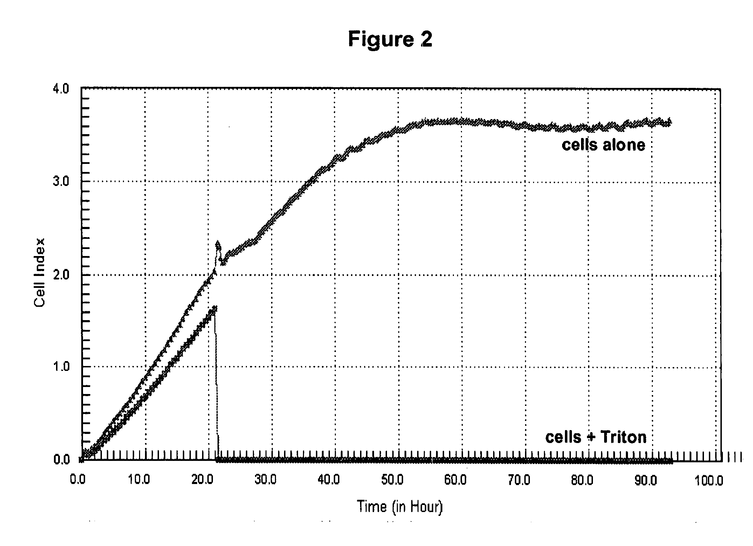

In subsequent experiments, SKBR3 cells were plated at 40K per well. After 20 hours, fresh media was added to one group of wells and PBS containing Triton X-100 was added to a second group of wells, and the plate was returned to the reader. As shown in Figure 2, a drop in Cell Index followed addition of Triton X-100, indicating cell death.

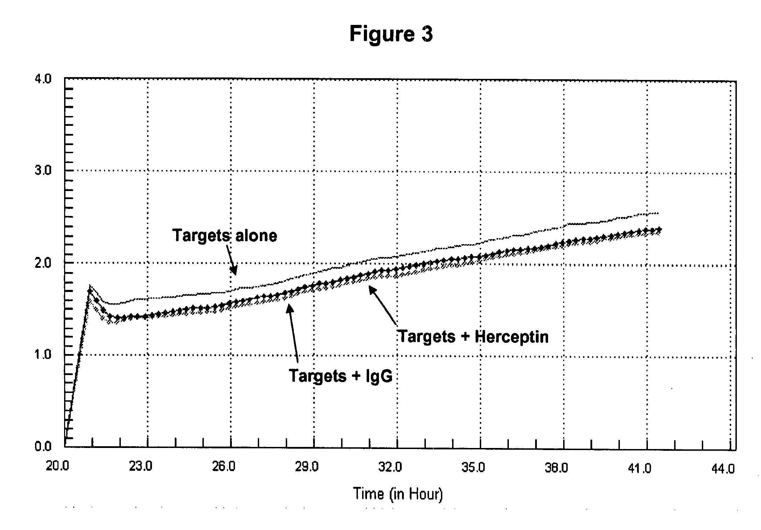

In further experiments, SKBR3 cells were plated at 20K per well, and media alone, Herceptin®, or IgG control antibody was added 20 hours later. The additional of either Herceptin® or IgG alone did not significantly alter the Cell Index compared to the Cell Index when no antibody was added (Figure 3).

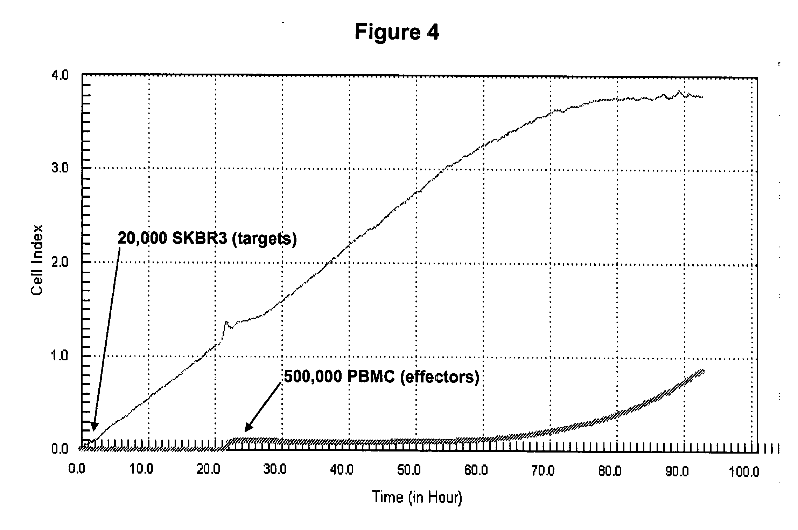

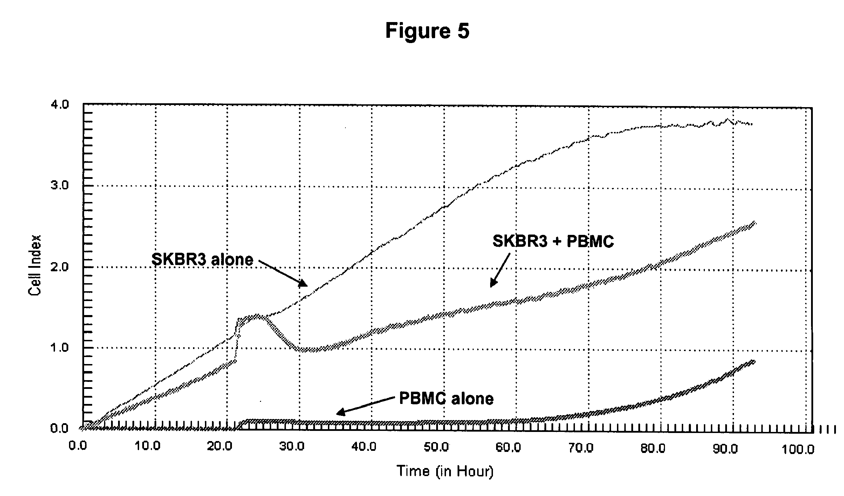

The Cell Index of SKBR3 cells plated at 20K per well was then compared with the Cell Index of PBMC added to media alone at a final amount of $5 \times 10^5$ cells per well. Surprisingly, PBMC did not have a significant effect on Cell Index (Figure 4), indicating that the use of PBMC as effector cells in an ADCC assay would not interfere with accurate detection of SKBR3 cell death. When PBMC were added to wells containing SKBR3 cells plated at 20K per well, the Cell Index dropped (Figure 5), indicating that PBMC effectors alone have an NK killing effect on SKBR3 target cells.

An assay was then conducted to detect ADCC killing of SKBR3 cells by the target antibody Herceptin® in the presence of PBMC effectors. Target cells were plated at 20K per well 20 hours before the start of the ADCC assay. At onset of the ADCC assay, the plate was removed from the ACEA System temporarily for addition of antibody and effector cells.

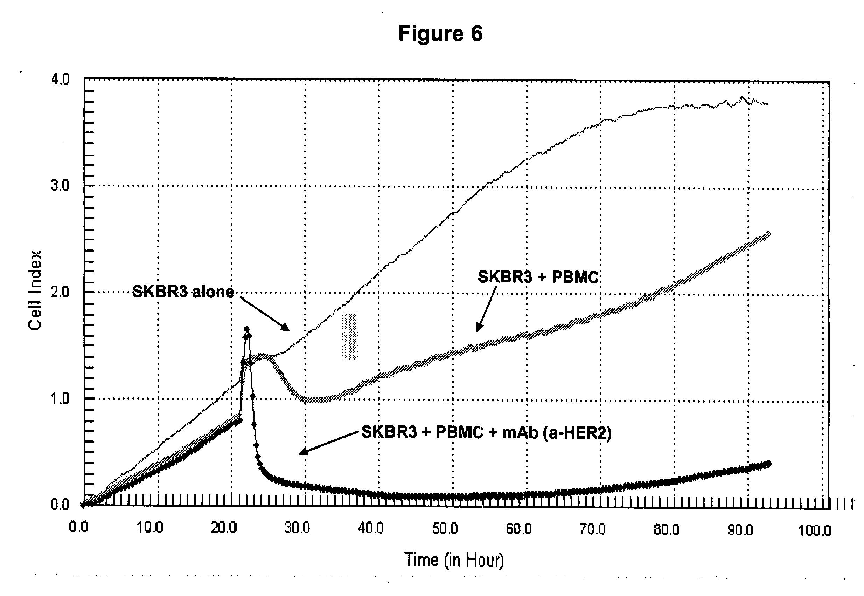

PBMC effector cells were added to the SKBR3 target cells at a ratio of 25:1effector:target, along with the Herceptin® antibody at a fmal concentration of 5 $\mu$g/ml. The cell plate was then returned to the ACEA System for continuous monitoring of Cell Index for the next 48 hours. Figure 6 shows a comparison of the Cell Index of SKBR3 cells alone, SKBR3 cells with PBMC, and SKBR3 cells with Herceptin® antibody and PBMC. A dramatic drop in Cell Index was recorded after additional of PBMC and Herceptin®, reflecting killing of SKBR3 cells by ADCC.

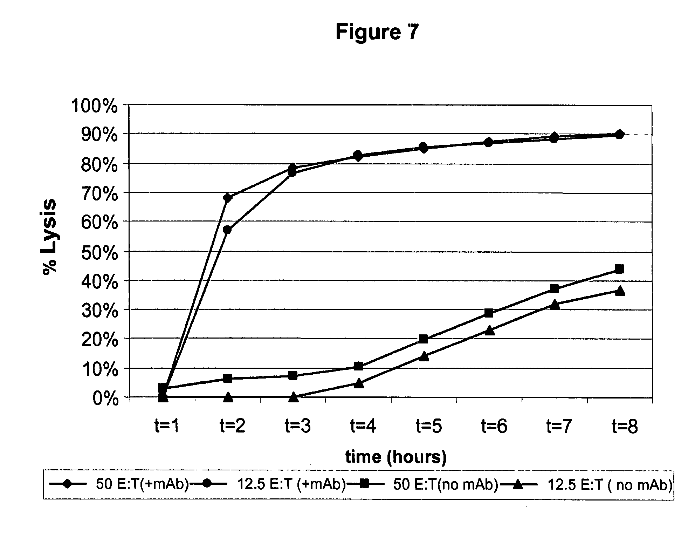

The experiment was repeated using SKBR3 cells with PBMC effectors at a 50:1 or 12.5:1 E:T ratio, with or without Herceptin® at a concentration of 5 $\mu$g/ml. Figure 7 shows a time-course of ADCC killing in these experiments as measured in terms of percent cell lysis, which was calculated according to the following equation:

$$\% \text{ lysis} = 100 \text{ X } \frac{\text{no antibody treatment Cell Index} - \text{antibody treatment Cell Index}}{\text{no antibody Cell Index}}$$

The results in Figure 7 demonstrate a dramatic increase in cell lysis in the presence of Herceptin® and PBMC as a result of ADCC.

*4. Comparison of real-time ADCC assay with Calcein AM Release ADCC assay*

Experiments were conducted to compare the % lysis detected using the real-time ADCC assay with the % lysis detected using a standard Calcein AM release ADCC assay.

The Calcein-AM assay was conducted using the following protocol. A vial of Calcein AM (Molecular Probes, Eugene, OR; catalog # C-3099) at 1 mg/ml solution in dry DMSO was thawed and mixed gently by pipetting.

To label the SKBR3 cells, the medium was removed and the monolayer was washed with 10 ml PBS alone (no fetal bovine serum (FBS)). The monolayer was detached by adding 5 ml EDTA/PBS and incubating up to 10 minutes at 37°C. Ten ml of fresh R10 (RPMI-1640 medium with 10% heat-inactivated FBS, Pen/Strep (final 100 $\mu$g/ml), and L-glutamine (final 2 mM)) was added. Cells were harvested, centrifuged, and counted, and 2.5 X $10^6$ cells were transferred to fresh 15 ml tubes. Cells were suspended in 2.5 ml of R-10 in a 15 ml conical tube, and 12.5 $\mu$l of Calcein AM (final 5 $\mu$M) was added to each tube. The tubes were incubated for 30 minutes at 37°C in humidified 5% $CO_2$, ensuring that the cap was loose. Cells were mixed gently every 10 minutes to avoid settling. Labeled cells were washed twice with 10 ml R-2 (RPMI-1640 medium with 2% heat-inactivated FBS). Cells were suspended R-10 medium to achieve 5 X $10^5$ cells/ml.

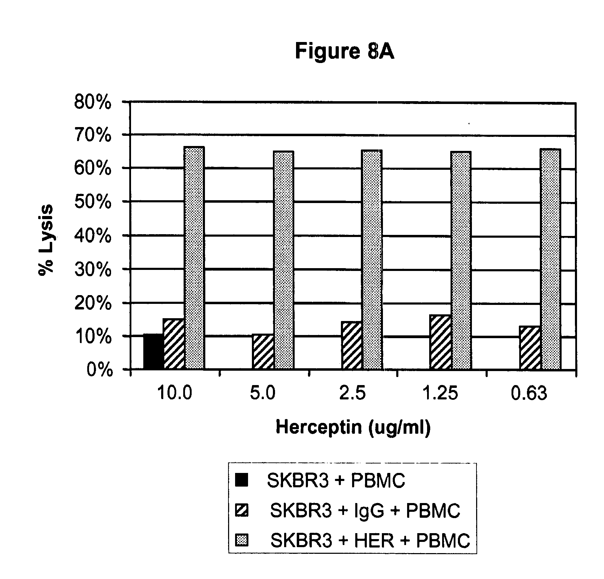

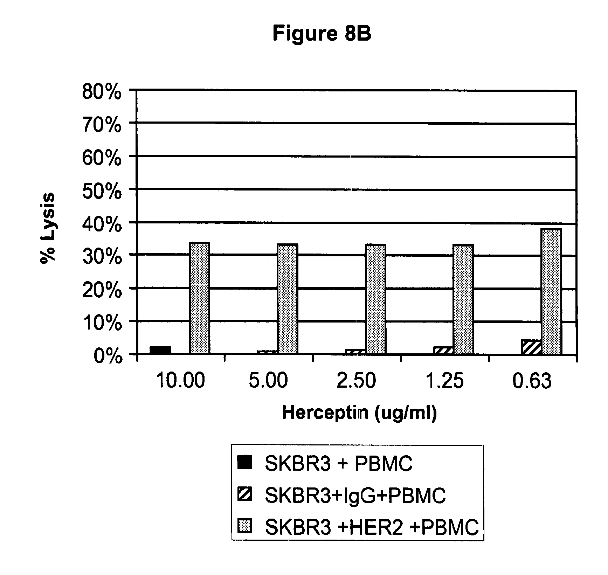

The target SKBR3 cells were plated at 20K per well for the ADCC assay. At onset of the ADCC assay, PBMC effector cells were added to the target cells at a ratio of 25:1, along with control antibody IgG or Herceptin® at the concentrations shown in Figure 8. The mixtures of target cells, effector cells, and antibodies were allowed to incubate at 37°C for four hours. At the end of the incubation, cells were pelleted and the release of Calcein AM was measured from the supernatant.

The average background media control for each target was subtracted for each individual target cell line prior to analysis, and the percent min to max was calculated using the following equation:

$$\% \text{ min to max} = 100 \text{ X } \frac{\text{mean spontaneous release cpm}}{\text{mean maximum release cpm}}$$

The assay was considered invalid if the percent of minimal release versus maximal release was more than 37%. The percentage specific lysis was calculated from the mean of triplicate points using the following equation:

$$\% \text{ lysis} = 100 \text{ X } \frac{\text{mean experimental cpm} - \text{mean spontaneous release cpm}}{\text{mean maximal release cpm} - \text{mean spontaneous release cpm}}$$

The percent lysis detected using the Calcein-AM assay is shown in Figure 8B. Figure 8A shows the percent lysis detected in a parallel assay conducted using the RT-CES® system. SKBR3 target cells were plated 18-20 hours prior to adding PMBC effector cells and IgG or Herceptin® antibodies in the same ratios and concentrations as for the Calcein-AM assay. The percent lysis detected using the RT-CES system was about 2-fold higher than the percent lysis detected using Calcein-AM labeling. This may be due to the fact that the Calcein-AM assay requires the cells to be in suspension, whereas the cells are adherent in the RT-CES assay.

*5. Assay of ADCC killing of MG63 cells by RX-1 monoclonal antibody and PBMC*

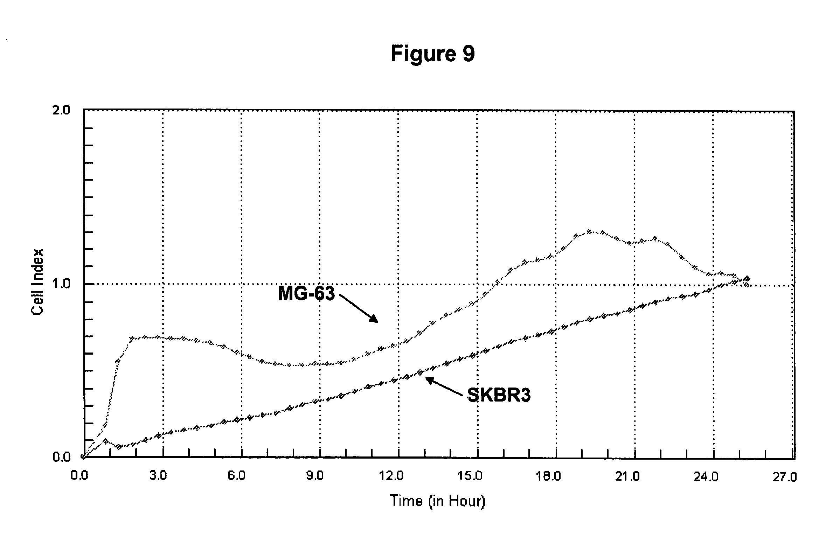

MG63 and SKBR3 cells were plated at 20K cells per well, and Cell Indices were monitored for 24 hours. Growth of SKBR3 cells had a linear relationship with the Cell Index, whereas growth of MG63 cells generally did not (Figure 9). Between about 7 hours and about 17 hours, however, MG63 cell growth was in an approximately linear relationship with Cell Index, suggesting that the ADCC assay should be performed within this time period for MG63 cells.

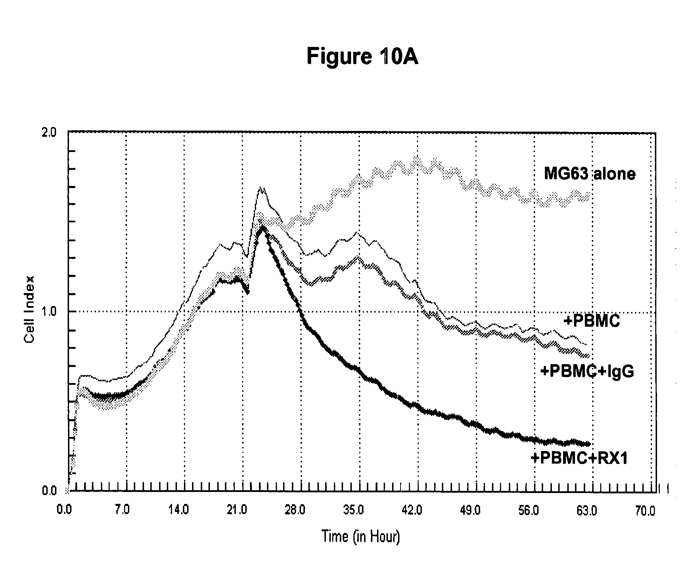

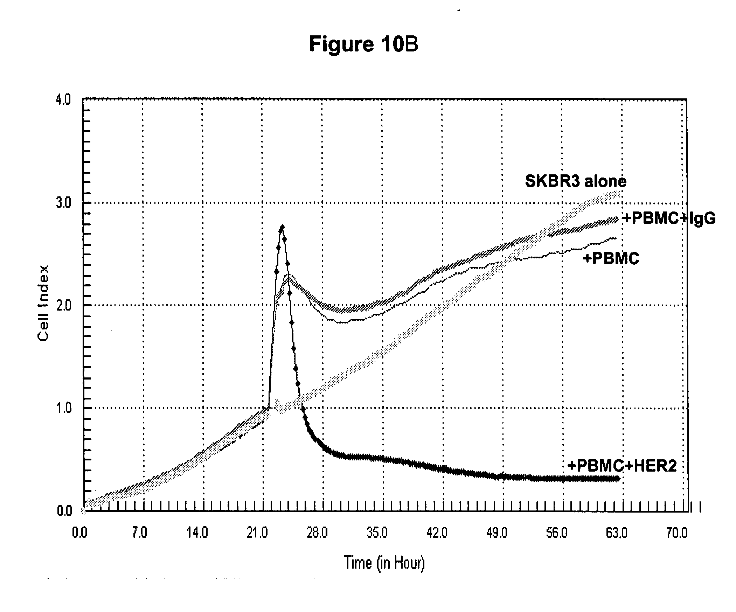

An ADCC assay was conducted with the MG63 target cells plated at 20K per well. The Cell Index for MG63 cells alone was compared with the Cell Index of MG63 cells to which PBMC, PBMC and IgG control antibody, or PBMC and RX1 antibody was added after 18-20 hours. The PBMC effector to MG63 target cell ratio in each experiment was 50:1, and the antibody was added at a concentration of 5 $\mu$g/ml. As shown in Figure 10A, the Cell Index fell following addition of PBMC and RX1 as a result of ADCC of the MG63 cells. The kinetics of the ADCC were, however, different from the kinetics observed when the same experiment was repeated with SKBR3 cells in the presence of PBMC, PBMC and IgG, or PBMC and Herceptin® (Figure 10B). These results demonstrate the utility of the real-time ADCC assay in providing detailed information on ADCC kinetics.

*6. Conclusion*

The ADCC assays described herein provide improvements over current methods for assaying ADCC. Unlike standard assays, the RT-CES®-based assays can be used to follow ADCC killing in real-time, and allow the kinetics of ADCC to be followed in detail. The assays are more sensitive than other ADCC assays that are currently available, are faster, and have the potential to be optimized for high-throughput screening. In addition, the assays are label-free and non-radioactive, or require reduced levels of label, making them safer than assays involving radioactive labeling, for example. Since the assays can be conducted on adherent cells, without detachment from the plate, they allow a more accurate assessment of ADCC on cells *in vivo.* Thus, the ADCC assays described herein will improve the ability to assay candidate therapeutic antibodies for ADCC activity.

Furthermore, the ADCC assays provided herein may be used to identify patient populations suitable for treatment with a particular antibody. As discussed above, the assays can be conducted using PBMCs as effector cells. Polymorphisms exist in the Fc receptors on PBMC effector cells that can affect the ability of the effector cells to bind a target-specific antibody, and thus to mediate ADCC. The ADCC assays provided herein are quick and easy assays that can be used to determine whether the PBMC from a particular patient, in combination with a therapeutic antibody, are capable of mediating ADCC of target cells.

In summary, the ADCC assay methods provided herein have the potential to change the way ADCC is measured, both in industry for drug development, and in basic research for studying immune response mediated by antibodies.

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

**1.** A method of assaying antibody-dependent cellular cytotoxicity (ADCC), comprising:

(a) monitoring the impedance between electrodes on a non-conducting substrate that supports the growth of target cells in an assay medium; and
(b) adding to the assay medium effector cells and an antibody that binds to the target cells;
wherein a decrease in the impedance between the electrodes on the substrate following addition of the effector cells and the antibody is indicative of ADCC function having been effected in the assay medium, and wherein an increase or no change in the impedance between the electrodes on the substrate following addition of the effector cells and the antibody is indicative of ADCC function not having been effected in the assay medium; wherein, preferably:

(i) the antibody is derived from a patient with an autoimmune disorder; or
(ii) the method comprises measuring impedance at regular intervals, wherein more preferably the method comprises deriving a Cell Index (CI) from each impedance measurement and determining whether a change in Cell Index occurs, wherein the Cell Index is derived from each impedance measurement using the formula

$$ CI = \max_{i=1,..,N}\left( \frac{R_{cell}(f_i)}{R_b(f_i)} - 1 \right) $$

wherein $R_b(f)$ and $R_{cell}(f)$ are the frequency-dependent electrode resistances without cells or with cells present, respectively, and $N$ is the number of the frequency points at which the impedance is measured.

2. The method according to claim 1, wherein the method is conducted using the RT-CES® system.

3. The method according to any one of claims 1 to 2, wherein:

(i) impedance measurements are taken every 15 minutes; or
(ii) the method further comprises plating the target cells and wherein, preferably, the target cells are plated 18 to 24 hours prior to addition of the antibody and effector cells; or
(iii) the target cells are in a monolayer on the substrate.

4. The method according to any one of claims 1 to 3, wherein the target cells are plated at a density of: (i) between 2K and 100K per well, (ii) between 15K and 25K per well or (iii) about 20K per well.

5. The method according to any one of claims 1 to 4, wherein the ratio of effector cells to target cells (E:T) is: (i) 25:1, (ii) greater than 10:1, (iii) greater than 50:1, or (iv) greater than 100:1.

6. The method according to any one of claims 1 to 5, wherein the antibody is added at a concentration of between: (i) about 1 and about 100 [mu]g/ml, (ii) about 1 and about 50 [mu]g/ml, or (iii) about 2 and about 8 [mu]g/ml.

7. The method according to any one of claims 1 to 6, comprising adding to the assay medium: (i) two or more antibodies that bind to the target cells; three or more antibodies that bind to the target cells; or (iii) four or more antibodies that bind to the target cells.

8. The method according to any one of claims 1 to 7, wherein the target cells express apical antigens, wherein, preferably, the method comprises a preliminary step of screening the target cells for apical antigen expression.

9. The method according to any one of claims 1 to 8, wherein the target cells are cancer cells or virally-infected cells, wherein, preferably: (i) the target cells are cancer cells; and/or (ii) the cancer cells are from a cell line; and/or (iii) the cancer cells are SKBR3 cells or MG63 cells.

10. The method according to any one of claims 1 to 9, wherein:

(i) the effector cells comprise peripheral blood mononuclear cells (PBMCs), natural killer (NK) cells, monocytes, cytotoxic T cells or neutrophils; or
(ii) step (b) comprises adding to the target cells whole blood that has been partially enriched for the effector cells; or
(iii) wherein step (b) comprises adding whole blood to the effector cells, and wherein the whole blood comprises the effector cells.

11. A method of screening a candidate antibody for the ability to induce ADCC against target cells, comprising:

(a) monitoring the impedance between electrodes on a non-conducting substrate that supports the growth of target cells in the assay medium; and
(b) adding effector cells and the candidate antibody that binds to the target cells to the assay medium; wherein a decrease in the impedance between the electrodes on the substrate following addition of the effector cells and the antibody is indicative of the ability of the candidate antibody to effect ADCC function against the target cells, and wherein an increase or no change in the impedance between the electrodes on the substrate following addition of the effector cells and the antibody is indicative of the inability of the candidate antibody to effect ADCC function against the target cells.

12. A method of identifying a patient having a disease associated with target cells that is suitable for treatment with a candidate antibody, the method comprising:

(a) monitoring the impedance between the electrodes on a nonconducting substrate that supports the growth of target cells associated with the disease;
(b) adding PBMCs isolated from the patient and the candidate antibody to the target cells; and
(c) determining whether a change in the impedance between the electrodes on the substrate occurs following addition of the PBMCs and the antibody, wherein a decrease in impedance between the electrodes is indicative of the patient's suitability for treatment with the antibody, and wherein an increase or no change in the impedance between the electrodes on the substrate following addition of the PBMCs and the antibody is indicative of the patient's lack of suitability for treatment with the antibody.

13. A method of screening a candidate compound for the ability to modulate ADCC, comprising:

(a) monitoring the impedance between electrodes on a non-conducting substrate that supports the growth of target cells in the assay medium;
(b) adding to the assay medium effector cells and an antibody, in the presence and absence of the candidate compound, wherein the antibody binds to the target cells; and
(c) comparing any change in the impedance between the electrodes on the substrate following addition of the effector cells and the antibody in the presence of the candidate compound with any change in the impedance between the electrodes on the substrate following addition of the effector cells and the antibody in the absence of the candidate compound, wherein a change in the impedance in the presence of the candidate compound that is greater than any change in the impedance in the absence of the candidate compound is indicative that the candidate compound has the ability to modulate ADCC; wherein, preferably, the compound to be screened modulates autoimmune - related ADCC.

14. A method according to any previous claim which is a high-throughput assay, and wherein the non-conducting substrate comprises two or more microtiter wells, each well comprising at least two electrodes, and monitoring the impedance between the electrodes in each well.

15. A quality control assay for an antibody, comprising:

(a) monitoring the impedance between electrodes on a non-conducting substrate that supports the growth of target cells in an assay medium; and
(b) adding effector cells and the antibody to the assay medium, wherein the antibody binds to the target cells; wherein a decrease in the impedance between the electrodes on the substrate following addition of the effector cells and the antibody is indicative that the antibody is suitable to be released for use in ADCC induction, and wherein an increase or no change in the impedance between the electrodes on the substrate following addition of the effector cells and the antibody is indicative that the antibody is not suitable to be released for use in ADCC induction.

16. A quality control assay for an antibody, comprising:

(a) monitoring the impedance between electrodes on a non-conducting substrate that supports the growth of target cells in an assay medium;
(b) adding effector cells and the antibody to the assay medium, wherein the antibody binds to the target cells; and
(c) comparing any change in the impedance between the electrodes on the substrate following addition of the effector cells and the antibody with any change in the impedance for a control sample following addition of the effector cells and a control antibody; wherein a decrease in the impedance following addition of the effector cells and the antibody that is greater than any decrease in the impedance for the control sample following addition of the effector cells and the control antibody is indicative that the antibody is suitable to be released for use in ADCC induction, and wherein lack of a decrease in the impedance following addition of the effector cells and the antibody that is greater than any decrease in the impedance for the control sample following addition of the effector cells and the control antibody is indicative that the antibody is not suitable to be released for use in ADCC induction; and

wherein, preferably a decrease in the impedance following addition of the effector cells and the antibody that is at least 25% greater than the decrease in the impedance for the control sample following addition of the effector cells and the control antibody is indicative that the antibody is suitable to be released for use in ADCC induction.

17. A method of screening a candidate compound for use as a therapeutic against an autoimmune disease, comprising:

(a) monitoring the impedance between electrodes on a non-conducting substrate that supports the growth of target cells in an assay medium, wherein the target cells are healthy cells;
(b) adding to the assay medium effector cells and an antibody, with and without the candidate compound, wherein the antibody binds to the target cells, and wherein the effector cells are PBMCs from a subject diagnosed with the autoimmune disease; and
(c) comparing any change in the impedance in the presence of the candidate compound with any change in the impedance in the absence of the candidate compound, wherein a decrease in the impedance in the absence of the candidate compound that is greater than any decrease in the impedance in the presence of the candidate compound is indicative that the candidate compound is suitable as a therapeutic agent against the autoimmune disease, and wherein lack of a decrease in the impedance in the absence of the candidate compound that is greater than any decrease in the impedance in the presence of the candidate compound is indicative that the candidate compound is not suitable as a therapeutic agent against the autoimmune disease.

18. A method of determining whether a candidate antibody is suitable for treating a subject having an autoimmune disease, comprising:

(a) monitoring impedance between the electrodes on a non-conducting substrate that supports the growth of target cells associated with the autoimmune disease; and
(b) adding the candidate antibody and PBMCs isolated from the subject to the target cells; and
(c) determining whether a change in the impedance between the electrodes on the substrate occurs following addition of the PBMCs and the candidate antibody, wherein a decrease in impedance between the electrodes is indicative that the antibody is suitable for treating the subject, and wherein the lack of a decrease in impedance between the electrodes is indicative that the antibody is not suitable for treating the subject.

19. A method for determining an optimal concentration of an antibody for inducing an ADCC response, comprising:

(a) monitoring the impedance between electrodes on a non-conducting substrate that supports the growth of two or more samples of target cells in an assay medium; and
(b) adding effector cells and an antibody to the two or more samples of target cells, wherein the antibody binds to the target cells, and wherein the antibody is added at different concentrations to the two or more samples of target cells; wherein a decrease in the impedance between the electrodes on the substrate following addition of the effector cells and the antibody is indicative of ADCC function having been effected in the assay medium, and wherein the concentration of antibody that results in the greatest decrease in impedance is determined to be the optimal concentration.

20. A method of determining whether an antibody binds to an apical antigen on a target cell, comprising:

(a) monitoring the impedance between electrodes on a non-conducting substrate that supports the growth of target cells in an assay medium; and
(b) adding effector cells and the antibody to the target cells; wherein a decrease in the impedance between the electrodes on the substrate following addition of the effector cells and the antibody is indicative that the antibody binds to an apical antigen on the target cells.

## Figure 1

EP 2 246 700 A1

Figure 2

EP 2 246 700 A1

Figure 3

Figure 4

EP 2 246 700 A1

## Figure 5

**Figure 6**

EP 2 246 700 A1

Figure 7

EP 2 246 700 A1

Figure 8A

Figure 8B

EP 2 246 700 A1

Figure 9

EP 2 246 700 A1

Figure 10A

EP 2 246 700 A1

Figure 10B

EP 2 246 700 A1

Europäisches
Patentamt

European
Patent Office

Office européen
des brevets

# EUROPEAN SEARCH REPORT

Application Number

EP 10 00 8288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | COMLEY J: "Label-Free Detection" DRUG DISCOVERY WORLD, R J COMMUNICATIONS & MEDIA WORLD LTD., LONDON, GB, 2004, pages 63-74, XP002404746 ISSN: 1469-4344 * page 72, column 1, line 1 - column 2, line 1 * * figure 1 * ----- | 1-20 | INV. G01N33/50 |
| A,D | WO 2005/047482 A (XU XIAO [US]; ABBASSI YAMA [US]; WANG XIAOBO [US]; GAN JIANGBO [US]) 26 May 2005 (2005-05-26) * claims 72,77,80-90 * ----- | 1-20 | |
| A | US 5 840 313 A (VAHLNE ANDERS [SE] ET AL) 24 November 1998 (1998-11-24) * example 6 * ----- | 1-20 | |
| A | EP 1 176 195 A1 (KYOWA HAKKO KOGYO KK [JP]) 30 January 2002 (2002-01-30) * page 25, line 41 - page 26, line 28 * ----- | 1-20 | |
| X,P | GLAMANN JOAKIM ET AL: "Dynamic detection of natural killer cell-mediated cytotoxicity and cell adhesion by electrical impedance measurements." ASSAY AND DRUG DEVELOPMENT TECHNOLOGIES OCT 2006, vol. 4, no. 5, October 2006 (2006-10), pages 555-563, XP002442508 ISSN: 1540-658X * the whole document * ----- -/-- | 1-20 | TECHNICAL FIELDS SEARCHED (IPC) G01N |

The present search report has been drawn up for all claims

| Place of search | Date of completion of the search | Examiner |
|---|---|---|
| The Hague | 21 September 2010 | van der Kooij, M |

Europäisches
Patentamt

European
Patent Office

Office européen
des brevets

**EUROPEAN SEARCH REPORT**

Application Number

EP 10 00 8288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2006/015387 A (XU XIAO [US]; ABASSI YAMA [US]; WANG XIAOBO [US]) 9 February 2006 (2006-02-09) * the whole document * * page 10, line 15 - line 29 * * page 11, line 28 - page 14, line 9 * * page 38, line 7 - line 9 * * page 119, line 12 - page 121, line 33 * ----- | 1-20 | |

TECHNICAL FIELDS
SEARCHED       (IPC)

The present search report has been drawn up for all claims

| Place of search | Date of completion of the search | Examiner |
|---|---|---|
| The Hague | 21 September 2010 | van der Kooij, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

               

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 10 00 8288

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2010

| Patent document cited in search report | | Publication date | Patent family member(s) | | Publication date |
|---|---|---|---|---|---|
| WO 2005047482 | A | 26-05-2005 | CA | 2550274 A1 | 26-05-2005 |
| | | | EP | 1692258 A2 | 23-08-2006 |
| US 5840313 | A | 24-11-1998 | NONE | | |
| EP 1176195 | A1 | 30-01-2002 | AU | 3672800 A | 14-11-2000 |
| | | | CA | 2369292 A1 | 19-10-2000 |
| | | | WO | 0061739 A1 | 19-10-2000 |
| | | | JP | 4368530 B2 | 18-11-2009 |
| | | | JP | 2009171976 A | 06-08-2009 |
| | | | JP | 2009275049 A | 26-11-2009 |
| | | | US | 2005272916 A1 | 08-12-2005 |
| WO 2006015387 | A | 09-02-2006 | CA | 2575297 A1 | 09-02-2006 |
| | | | CA | 2575573 A1 | 16-02-2006 |
| | | | EP | 1773979 A2 | 18-04-2007 |
| | | | EP | 1773980 A2 | 18-04-2007 |
| | | | WO | 2006017762 A2 | 16-02-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 75630106 P
- WO 2004010103 A
- WO 2004010102 A
- WO 200577104 A
- WO 2005047482 A
- US 20050112544 A
- WO 2005077104 A

**Non-patent literature cited in the description**

- **Nelson et al.** *Nature,* 2003, vol. 422, 766-774
- **Snijdewint et al.** *Int. J. Cancer,* 2001, vol. 93, 97-106
- **Hayes et al.** *J. Immunol.,* 1990, vol. 145, 962-970
- **Lu et al.** *Cancer Immunol. Immunother.,* 2002, vol. 51, 153-162